# EUROPEAN PATENT APPLICATION

(11) **EP 3 050 530 A1**
(43) Date of publication of application: **03.08.2016**
(21) Application number: 16275004.6
(22) Date of filing: 05.01.2016
(51) Int. Cl.: A61B 18/04, A61B 18/08, A61B 18/14, A61B 18/24, A61B 18/00, A61B 18/18, A61B 90/00

(54) **ABLATION APPARATUS WITH THROMBUS DETACHMENT MECHANISM**

(30) Priority: 16.01.2015 GB 201500733
(71) Applicant: Cook Medical Technologies LLC, Bloomington, IN 47402 (US)
(72) Inventor: Elgaard, Per, 4690 Haslev (DK); Torp, Allan, 4632 Bjaeverskov (DK); Paamand, Rune T., 2720 Vanloese (DK)
(74) Representative: Williams Powell

(57) **Abstract**

RF ablation apparatus (10) includes an anode electrode assembly (12) including an anode terminal (20) housed within a catheter (30). The catheter (30) includes a distal end wall (34) which is disposed in a plane substantially perpendicular to the longitudinal dimension of the catheter (30). The anode electrode (22) is slidably received within the cathode (30) and can be extended out beyond the distal end (32) of the catheter (30) to provide at least a first terminal for an ablation circuit. The distal end wall (34) is sized and shaped to hold a thrombus formed on the anode electrode (22) so that the anode electrode (22) can be withdrawn from within the thrombus formation and into the catheter (30), without the thrombus formation being pulled with the anode terminal (22). The outer surface of the catheter (30), or at least at the distal end (32) thereof, is made of an insulating or non-conducting material, such that a thrombus does not form over the catheter (30). The assembly allows for the generation of a thrombus to occlude a vessel (60) and without the risk of pulling any formed thrombus with the apparatus upon retrieval of the apparatus from the patient.

## Description

### Technical Field

The present invention relates to medical ablation apparatus and to a method of ablating a vessel of a patient. The preferred embodiments are directed to an RF ablation apparatus but the disclosure herein extends to ablation by other mechanisms, including for example, resistive heating systems, laser and microwave heating systems.

### Background Art

There have been various studies on the development of RF vessel ablation techniques for closing a patient's blood vessel and a number of devices developed to this end. There are currently two principal systems under consideration, the first being a monopolar system in which an electrode is placed in a patient's vessel at the point of desired ablation and a return conductor generally in the form of a large surface area pad which is positioned against the patient's body at a location closest to the embedded electrode. Electrical current is passed through the electrode pair at RF frequencies, with the patient's body closing the circuit path between the two electrodes.

Another system, generally known as a bipolar system, provides both feed and return electrodes at the distal end of an elongate carrier which is positioned in a patient's vessel to be ablated. A bipolar system of this type is also fed with electrical current at RF frequencies.

There are various designs of system of these types under consideration for effecting vessel ablation. They generally seek to ablate either the vessel walls or blood within the vessel or both. Ablation of a vessel wall causes the vessel to collapse or constrict, thereby to close off the vessel. Ablation of the blood produces a blood clot intended to occlude the vessel.

A number of difficulties have been encountered with systems developed to date, including potential damage to the vessel wall or surrounding organs and tissue, and potential opening of the vessel or loss of occlusion during removal of the endoluminal electrode. Loss of occlusion can occur as a result of sticking of any formed thrombus to the endoluminally placed electrode and as a consequence of the thrombus being pulled from the treated vessel site as the electrode is withdrawn. These problems can be experienced with both monopolar and bipolar systems.

Examples of ablation apparatus can be found in US-8,500,731, US-5,403,311, US-5,536,267, US-2011/0,301,594, US-6,090,105, US-8,734,439 and US-5,411,025.

### Disclosure of the Invention

The present invention seeks to provide improved apparatus for and method of ablating a patient's vessel.

According to an aspect of the present invention, there is provided medical ablation apparatus including: an endoluminal delivery catheter having at least one lumen therein, the lumen having a transverse size and cross-sectional shape, the catheter having an outer surface and a distal end wall, the outer surface extending to the distal end wall; and an elongate flexible ablation device having a transverse size and cross-sectional shape substantially conforming to the transverse size and cross-sectional shape of the catheter lumen, the elongate ablation device having a blunt distal end, the elongate ablation device being slidably received in the catheter; an adjustment device for retracting the elongate ablation device into the catheter; wherein at least the distal end wall of the catheter is isolated from the elongate ablation device and provides a rigid insulated detachment shoulder.

In the preferred embodiment, the elongate flexible ablation device is unable to penetrate into the tissue of a vessel wall, that is has a flexibility which is greater than the resistance of the vessel wall to puncturing. The provision of a blunt distal end, which may be rounded for example, will ensure that the device does not have any characteristic which could cause the device to pierce into the vessel wall during normal operating conditions.

In an embodiment, the apparatus is an RF ablation apparatus and the elongate ablation device includes at least one electrically conductive terminal forming part of an electrical circuit of the ablation apparatus; wherein at least the distal end wall of the catheter is isolated from the elongate ablation device and provides an electrically insulated detachment or stop shoulder.

The electrically conductive terminal is preferably a flexible wire.

In other embodiments, elongate ablation device includes a resistive heating element, a heat conductive probe, such as a hot copper wire, an optical fibre for delivery laser energy, a microwave antenna, or other heating mechanism.

The detachment or stop shoulder, in combination with the ability of the elongate ablation device to slide in the catheter, enables the removal of the elongate ablation device from within a formed thrombus and in such a manner in which it is possible to avoid dislodging the thrombus from the vessel, thereby ensuring that the thrombus remains in place. The insulating nature of the distal end of the catheter can ensure that none of the thrombus mass created during the ablation process grows over the catheter, thereby ensuring that the catheter is not attached to the thrombus or embedded therewithin. The catheter can therefore be retracted without pulling the thrombus with it.

Advantageously, the catheter lumen and the elongate ablation device are substantially circular in transverse cross-section.

Preferably, the transverse size of the lumen is no more than 0.1 millimetres, more preferably no more than 0.025 millimetres, greater than the transverse size of the elongate ablation device. In other words, the elongate ablation device is a close fit within the lumen of the catheter, which in practice can ensure that any thrombus formation attached to the terminal is scraped off the terminal.

In a practical embodiment, the distal end wall of the catheter has a diameter of at least 0.7 millimetres. The distal end wall of the catheter advantageously extends by at least 0.16 millimetres from the lumen to the outer surface of the catheter. Preferably, the distal end wall of the catheter has a diameter at least two times the diameter of the electrically conductive terminal. The distal end wall advantageously is of a size sufficient to act as a stop shoulder for pushing any thrombus formation off the elongate ablation device.

In some embodiments there may be provided a length of insulation sleeve over a part of the electrically conductive terminal of the elongate ablation device, disposed adjacent the end wall of the catheter. The insulation sleeve can ensure that there is electrical and thermal isolation between the conductive terminal and the sleeve, further reducing any chance of growth of thrombus over the catheter.

In the preferred embodiment the catheter is entirely electrically insulating at all exposed surfaces thereof. The catheter is advantageously also thermally insulating.

In an embodiment, at least the distal end of the catheter is electrically non-conducting. It may be made of conductive material, such as metal, but electrically isolated from the ablation circuit, that is from the or each conductive terminal of the apparatus.

In another embodiment, at least the end wall is made of an insulating material. It may, for example, be made of polytetrafluoroethylene (PTFE), or a suitable polymer having a melting point of at least 200 degrees Centigrade.

In another embodiment, the distal end wall includes an inflatable balloon.

Advantageously, the distal end wall of the catheter is radiopaque. This may be by use of radiopaque materials in one or more of the distal end elements or by means of a radiopaque band, for instance.

In the preferred embodiment, the catheter and electrical terminal form part of a monopolar ablation device. Another embodiment provides a bipolar device, with both electrical terminals being able to be withdrawn into the catheter.

According to another aspect of the present invention, there is provided a method of ablating a vessel of a patient by means of medical ablation apparatus including an endoluminal delivery catheter having a lumen therein, the lumen having a transverse size and cross-sectional shape, the catheter having an outer surface and a distal end wall, the outer surface extending to the distal end wall, at least the distal end wall being insulating; and an elongate ablation device having a transverse size and cross-sectional shape substantially conforming to the transverse size and cross-sectional shape of the catheter lumen, the elongate ablation device being slidably received in the catheter; wherein the distal end wall of the catheter provides an insulated detachment or stop shoulder; the method including the steps of:
inserting the catheter endoluminally into a blood vessel of a patient to a treatment site;
applying energy to the elongate ablation device to effect ablation, whereby blood in the vessel coagulates around the elongate ablation device;
retracting the elongate ablation device into the lumen of the catheter with the distal end wall positioned adjacent the coagulated blood, whereby the coagulated blood is held in position by the distal end wall during the ablation device retraction step.

Preferably, the insulating end wall avoids the generation of a blood clot over the catheter.

Other features and advantages of the system and method taught herein will become apparent from the specific description which follows.

### Brief Description of the Drawings

Embodiments of the present invention are described below, by way of example only, with reference to the accompanying drawings, in which:
Figure 1 is a schematic diagram of an embodiment of RF ablation apparatus according to the teachings herein;
Figure 2 is a schematic side elevational view of an embodiment of endoluminal conductive electrode for the apparatus of Figure 1;
Figure 3 is a schematic side elevational view of another embodiment of endoluminal conductive electrode;
Figure 4 is a schematic side elevational view of yet another embodiment of endoluminal conductive electrode;
Figures 6 to 10 are schematic elevational views of an ablation process using the electrode of Figure 3 in occluding a patient's vessel;
Figure 11 shows another embodiment of anode electrode assembly according to the teachings herein;
Figure 12 is a schematic side elevational view of an embodiment of endoluminal conductive electrode for a bipolar RF ablation system; and
Figure 13 shows the distal end of another embodiment of catheter for the apparatus taught herein.

### Description of the Preferred Embodiments

The accompanying drawings are schematic only. It is to be understood that the dimensions and proportions of the various components of the apparatus shown in the drawings are not to scale or in proportion relative to one another. It is also to be understood that the drawings depict only the principal components of the apparatus shown therein and that other elements normally found in such apparatus which are not central to understanding the teachings herein have been omitted for the sake of clarity.

The principal embodiments described herein are directed to a monopolar RF ablation system in which one electrode of the system is located within the vessel to be occluded and the other electrode of the apparatus is kept outside the patient and typically in the form of a large surface area conductive pad which can be placed against the patient's skin at a location closest to the vessel to be treated. As is described below, though, the teachings herein can also be used for a bipolar system, in which both electrodes of the apparatus are disposed within the vessel to be occluded and thereby omitting any external electrode from the system. The skilled person will appreciate the relative merits of the two different types of system, which have been reported in the art.

It is furthermore to be understood that although the preferred embodiments disclosed herein are in the form of RF ablation devices, the teachings herein are not limited to this type of ablation. The mode of ablation, ids fact, is not relevant to the teachings herein. Other examples include resistive, optical, electromagnetic systems, in which, for example, the elongate ablation device may include a resistive heating element, a heat conductive probe, such as a hot copper wire, an optical fibre for delivery laser energy, a microwave antenna or other heating element. As the structures of such apparatuses will be immediately evident from the teachings herein, specific examples of these alternatives are not described in detail.

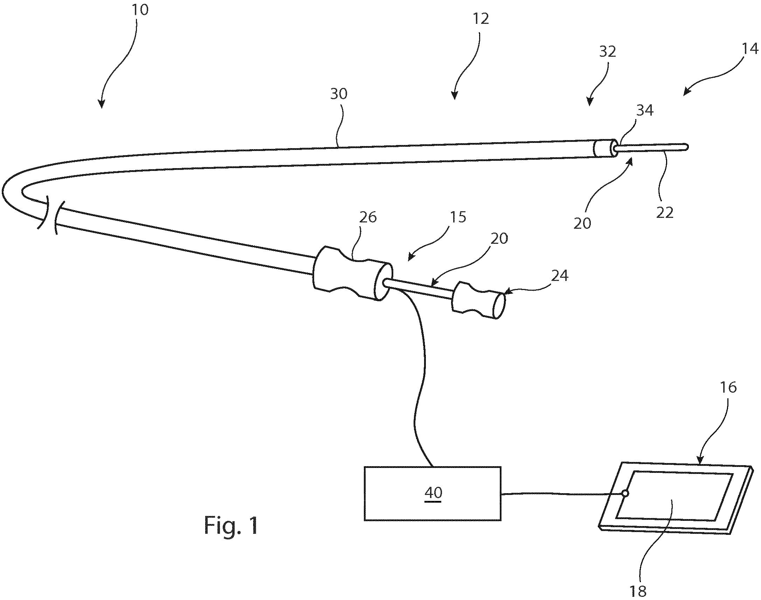

Referring first to Figure 1, this shows a schematic diagram of the principal components of an embodiment of monopolar RF vessel ablation apparatus 10. The apparatus 10 includes an anode electrode structure 12 which is of elongate form and of a length sufficient to be able, via endoluminal placement from a remote percutaneous entry point, to position the distal end 14 of the anode structure 12 at the site of a vessel to be occluded, with the proximal end being kept outside the patient. The electrode structure 12 may typically have a length from a few tens of centimetres to a metre or more, although its length is not material to the teachings herein.

The apparatus 10 also includes a second electrical terminal 16 which in this embodiment is in the form of a conductive terminal pad 18, constituting the cathode electrode of the RF ablation circuit. The conductive terminal pad 18 may be flexible so as to conform to the contours of the patient's body at the point at which it is positioned. In other embodiments, the conductive pad 16 may be rigid and optionally pre-shaped to the patient's contours as desired.

The anode electrode structure 12 includes an elongate electrode 20 having a distal tip 22 and an electrical conductor extending from the distal tip 22 internally through a lumen of the catheter 30 to a proximal end 15 of the electrode structure 12. The elongate electrode 20 may be of uniform structure throughout its length. In other embodiments, it may have a different structure for the distal tip 22, for example to be more rigid, and a more flexible structure along the remainder of its length, to enhance the flexibility of the electrode 20 for passage through tortuous vasculature. The elongate electrode 20 includes at the proximal end 15 a handle 24 for gripping by the clinician during the medical procedure and for purposes which are described below. The handle includes one or more gripping elements, attached to the tip 22, enabling the physician to move the tip relative to the distal end of the insulating catheter, including to withdraw the tip 22 at least partially into the catheter. The skilled person will be aware of suitable mechanisms from those commonly available in the art.

The distal tip, which may in one example be a wire, is flexible and has a blunt end, so as to be unable to penetrate into the tissue of a vessel wall, that is it has a flexibility which is greater than the resistance of the vessel wall to puncturing. The provision of a blunt distal end, which may be rounded for example, will ensure that the device does not have any characteristic which could cause the device to pierce into the vessel wall during normal operating conditions. In practice, the tip 22 may curve with the curvature of the vessel wall of of other elements of the introducer assembly.

The elongate electrode 20 is disposed within a catheter 30 which in the preferred embodiment is constructed to be insulating at least at its outermost surface, typically by being made of insulating material including but not limited to: polyurethane (PU), polyethylene terephthalate (PTFE), silicon, nylon or any other suitable material. The catheter 30 includes at its distal end 32 a distal end wall 34, described in further detail below. At the proximal end 15 of the assembly 10, the catheter 30 is provided with a handle 36 for use by the clinician and also described in further detail below. The catheter 30 and its lumen 38 typically have a round transverse cross-section and in the preferred embodiment the electrode 20 is similarly round in cross-section.

The apparatus 10 includes a control unit 40 which is coupled to the anode electrode 20 and to the cathode terminal pad 18. The control unit 40 is responsible for supplying electrical energy at RF frequencies to the electrodes 20 and 18 and also for controlling the supply of energy, typically on the basis of one or more feedback measurements, such as sensed current drain, sensed temperature, time and so on. Suitable control parameters for RF ablation apparatus have been reported extensively in the art and are therefore familiar to the skilled person and are not described in further detail herein. As the apparatus taught herein can be used with any of the currently disclosed or otherwise suitable control parameters, the structure of the control unit 40 will be immediately apparent to the skilled person and is therefore not described in detail. The unit 40 will typically include a current supply, one or more sensors coupled to suitable probes, a user input and a processor, among other conventional components.

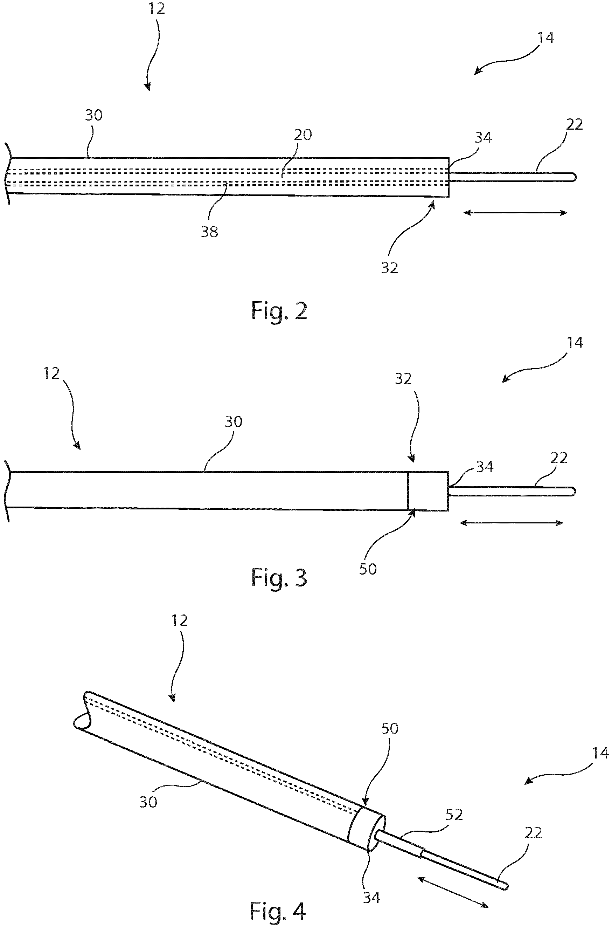

Referring next to Figure 2, this shows a first embodiment of anode electrode structure 12 and specifically the distal end 14 thereof. The catheter 30 includes a lumen 38 extending therethrough and within which the elongate electrode 20 can reside. The lumen 38 has a cross-sectional shape and size which conforms substantially to the cross-sectional shape and size of the electrode 20 and tip 22, in the preferred embodiment both being round in transverse cross-section. It is preferred that the electrode 20 and its tip 22 are a close fit within the lumen 38 of the catheter 30, with the lumen preferably being no more than around 0.1 millimetres greater than the transverse dimension, typically diameter, of the electrode 20, more preferably no more than 0.05 millimetres and most preferably no more than 0.025 millimetres greater. By having a close fit of these two components there is practically no gap between the electrode 20 (and its tip 22) and the internal wall of the catheter 30 which forms the lumen 38. In practice this ensures that no or virtually no material can enter the catheter 30 between the electrode 20 and the internal walls of the catheter 30.

The electrical tip 22 may be made of bare metal, although in other embodiments may be coated with a low frictional conductive material.

The catheter 30 may be made of a single material, typically the materials indicated above, although in some embodiments the catheter 30 may include one of more strengthening elements, such as braiding or a coil, of types known in the art.

The distal end wall 34 of the catheter 30 is preferably disposed in a plane perpendicular or substantially perpendicular to the longitudinal dimension of the catheter 30. It is preferred that the distal end wall 34 has a diameter which is at least two and a half (2½) times greater than the diameter of the electrode terminal 22, although in some embodiments the difference may be slightly less, such as two times. The actual dimensions of the catheter and in particular the diameter of the end wall 34 would be dependent upon the overall dimensions of the electrode assembly 12. It is preferred that the distal end wall of the catheter has a diameter of at least 0.7 millimetres, although could be substantially more than this. In some embodiments, particularly for ablation of small diameter vessels, the thickness of the distal end wall 34, that is between the internal surface forming the lumen 38 and the external surface of the catheter 30, is at least 0.16 millimetres.

In the embodiment of Figure 2, the catheter 30 is entirely electrically insulating at all of its exposed surfaces, typically being made of a non-conductive polymer material and having any strengthening elements, when these are provided, entirely embedded within the walls of the material forming the catheter. It is not excluded, however, that the catheter 30 could have one or more portions which are made of electrically conducting material, but in which case any such conducting material is isolated from the electrical circuit of the RF ablation apparatus 10.

It is common to form endoluminal catheters with soft tips to improve the trackability of the catheter in the patient's lumen and to made the distal end atraumatic. In the preferred embodiments disclosed herein, at least the trip of the catheter is harder than conventional catheter tips, in order to allow the catheter to act as a pusher element. This can be by making the entire catheter stiffer (with a higher durometer) at its distal end or by having the tip of a harder material (of higher durometer), in which case the catheter may have a softer (lower durometer) portion close to the distal tip for improved trackability.

Referring now to Figure 3, this shows another embodiment of anode electrode assembly 12 which differs from the embodiment of Figure 2 only at the distal end 32 of the catheter 30. As depicted in Figure 2, the electrode assembly shown has a distal end element 50 attached to the main portion of the catheter 30 and which provides the distal end wall 34. The distal element 50 may be made of an electrically insulating material and preferably one which has good tolerance to high temperatures. An example is polytetrafluoroethylene (PTFE) having a melting point of a327 degrees Centigrade or so. In another embodiment, the distal element 50 is made of a conductive material, such as a metal or metal alloy, but which is not coupled to the electrical circuit of the ablation apparatus. Typically, this can be achieved by isolating the distal element 50 from the electrical circuit. The distal element 50 could have a length of around 2 millimetres, up to a few millimetres.

Referring now to Figure 4, this shows an embodiment of anode electrode assembly 12 having a catheter structure 30 similar to that of the embodiment of Figure 3. It differs by the electrode 20 being partially covered in an insulating sleeve or coating 52. The sleeve or coating 52 may extend over the entire length of the electrode 20 apart from at the distal tip 22 and at the coupling to the conductor leading to the control unit 50. In other embodiments the sleeve or coating 52 may be disposed solely in the region of the distal end 32 of the catheter 30. The insulating sleeve or coating 52 provides electrical isolation between the distal element 50 and the conductive tip 22 of the electrode 20. It is specifically useful in embodiments where the distal element 50 is made of conducting material.

The sleeve or coating 52 is preferably bonded or otherwise attached to the electrode 20, although other embodiments may have a sleeve 52 which is attached to the catheter, with the electrode 20 being slidable within the sleeve.

In all of the embodiments of electrode assembly 12 shown in Figures 2 to 4, the electrode 20 is slidable within the catheter 30, in the direction of the arrows shown in these Figures. Thus, the distal tip 22 of the electrode 20 can be slid out from the distal end 32 of the catheter 30 and also retracted into the lumen 38 of the catheter 30, as described in further detail below.

The distal end 32 of the catheter may be made radiopaque, for example by means of a radiopaque band, by use of radiopaque materials, for example at the distal element 50, or by the incorporation of radiopaque elements within the material of the catheter 30 or distal element 50.

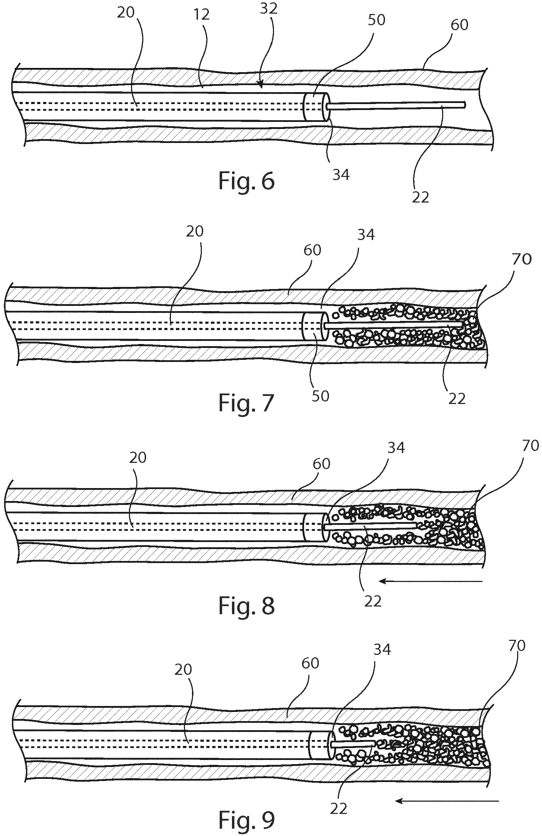

Figures 6 to 10 show the apparatus taught herein in use. Referring first to Figure 6, this shows the distal end 32 of the anode terminal apparatus 12 disposed within a vessel 60, specifically at a location in the vessel 60 at which it is desired to occlude the vessel by means of RF ablation. The electrode 20 can be seen with its conductive tip 22 exposed beyond the distal end 32 of the catheter 20. When electrical current is supplied by the control unit 50 of the apparatus 10 to the electrode 22, this will form a circuit with the cathode pad 16, through the patient's body. The relatively small surface area of the anode tip 22 will cause the blood within the vessel to heat, promoting the clotting of blood so as to form a thrombus 70, as shown in Figure 7. As the distal end wall 34 of the catheter 30 is isolated from the electrical circuit, the thrombus 70 forms around the anode tip 22 but does not extend over the catheter 20.

Once it has been determined that a thrombus has formed, for instance by measuring a change in temperature at the anode terminal 12, by determining a change in resistance in the circuit, or the like, the anode tip 22 can be retracted into the catheter 20, in the direction of the arrows shown in Figures 8 and 9. The control unit 50 may cease to supply electrical current to the RF ablation circuit at this time or may continue to apply current in order to continue causing blood clotting to enhance the occlusion. The distal end wall 34 of the catheter 20 acts as a stop shoulder preventing the thrombus 70 from moving as the tip 22 is retracted within the lumen 38 of the catheter 20. More specifically, the thrombus 70 is typically attached to the tip 22 as the result of the heat generated at the tip 22 but the tip 22 can nevertheless be pulled out from within the thrombus 70, with the distal end wall 34 acting to keep the thrombus 70 in position within the vessel and without any significant migration of the thrombus 70. For this purpose, the detachment shoulder is preferably rigid.

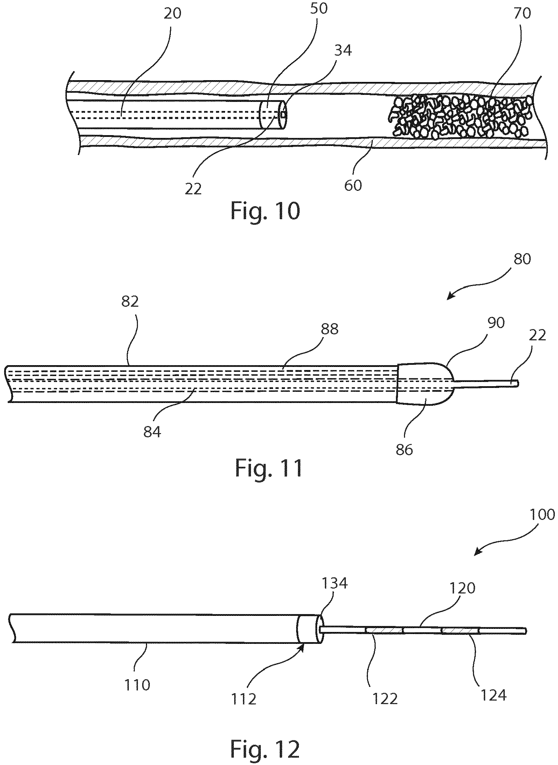

As shown in Figure 10, the distal end 22 of the electrode 20 can be retracted completely into the lumen 38 of the catheter 20, allowing the catheter 20 to be withdrawn from within the vessel 60, leaving the thrombus 70 in place.

Even in embodiments where the distal element 50 is made of a conducting material, such as metal or metal alloy, this is preferably of a volume which does not cause notable current through the element 50 and therefore no noticeable heating of the element 50.

The assembly 10, therefore, can occlude a vessel by RF ablation and in a manner in which the formed thrombus remains reliably in position within the vessel 60 without risk of dislodging the thrombus 70 as a result of its attachment to the electrode element 22, as can occur with the prior art structures.

Referring now to Figure 11, this shows another embodiment of anode electrode assembly 80 having characteristics similar to those of the earlier described embodiments. The assembly 80 includes a catheter 82 having a first lumen 84 therein into which the anode electrode 20 can be disposed such that its distal tip 22 can extend out of the catheter 82 and is slidable therewithin, in a manner similar to the previously described embodiments. The lumen 84 has the same characteristics as the lumen 38 of the earlier described embodiments.

At the distal end of the catheter 82 there is provided an inflatable balloon 86, which can be inflated and deflated via a second lumen 88 in the catheter 82, which is coupled to a source of inflation fluid such as saline solution or the like. The balloon 86 has a distal end 90 providing a stop shoulder or wall similar to the distal end surface 34 of the previously described embodiments. For this purpose, the balloon 86 may have a substantially truncated distal end 90 providing when inflated a surface in a plane substantially perpendicular to the longitudinal to the axis of the catheter 80. In other embodiments, there may be provided a support plate or disk attached to the distal end 90 of the balloon 86, possibly made of a polymer material such as PTFE or any of the other materials or structures described above.

The provision of a balloon 86 the distal end of the catheter 80 can increase flexibility of the assembly during deployment, when the balloon 86 will be deflated.

The structure is operated in the same manner as the earlier described embodiments, with the additional step of inflating and deflating the balloon 86 at the appropriate points in the procedure.

The catheter 80 may have the same structure and be made of the same materials as in the other described embodiments.

Referring now to Figure 12, this shows another embodiment of apparatus, in the form of a bipolar device. The assembly 100 includes a catheter 110 having the same characteristics as the catheters previously described and in the example shown having a distal element 112 similar to the distal element 50. The assembly 100 includes an elongate terminal 120 which includes first and second conductive electrodes 122, 124 which provide the cathode and anode terminals for the RF ablation circuit. Disposed within the electrode structure 120 are electrical wires or conduits (not shown) coupled at one end to the electrodes 122 and 124 and at the other end to a control unit similar to the control unit 40 of Figure 1. The bipolar electrode structure of the embodiment of Figure 12 avoids the need for an external electrode pad 16 of the type shown in Figure 1 and provides all of the heating energy and circuit within the vessel itself.

The embodiment of Figure 12 is broadly similar to the previously described embodiments, save for the provision of the dual electrodes 122, 124 at the electrode tip. Therefore, the disclosures above in connection with the other described embodiments apply equally to the embodiment of Figure 12.

When an RF current is passed through the electrodes, the heat generated in the blood by the current will cause a thrombus to be formed around the distal tip 120 and specifically around the anode and cathode electrodes 122, 124. The catheter 110 has a distal end wall 134 equivalent to the distal ends 34 of the earlier described embodiments and against which the thrombus can be held while the electrode assembly 120 is withdrawn into the catheter 110 after the ablation process, thereby to hold the thrombus in position within the vessel.

The person skilled in the art will appreciate that use of an electrically non-conducting end wall to the catheter 30, 82, 110 will avoid the generation of any blood clot over the catheter and as a result will allow the catheter to be removed without disturbing any formed thrombus.

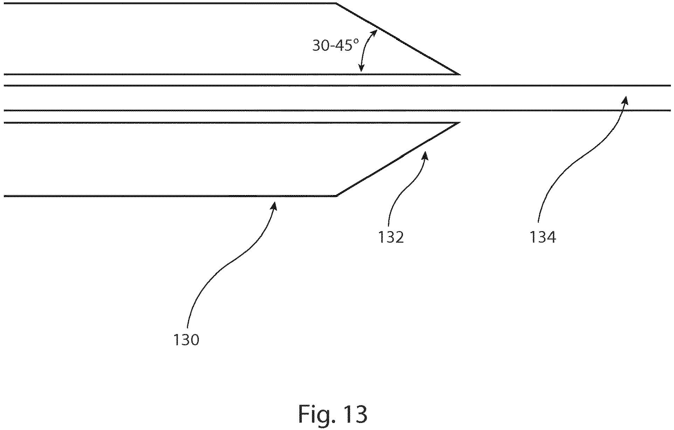

Referring now to Figure 13, this shows another embodiment of catheter 130 having a tapering tip 132. The tapering tip 132 may act as a wedge to remove thrombus material from the elongate ablation device 134. A suitable taper angle could be from 30° to 45°:

The structures described above have a number of advantages including, as described above, that of maintaining the thrombus in place after it has been formed. The apparatus also allows the size of the thrombus and therefore of the occluding barrier to be determined, by adjusting the exposed length of the anode electrode 22. In other words a longer thrombus formation can be formed by extending a greater length of electrode terminal beyond the distal end of the catheter, whereas a smaller thrombus formation can be formed by exposing only a short length of the electrode tip 22.

The apparatus 10 can be readily modified to enable a surgeon to adjust the exposed length of the conductor 22, for example by providing scale markings on the handle assembly 24, 26.

The close fit of the electrode 22, 120 within its respective catheter lumen ensures that no part of the formed thrombus can enter the lumen of the catheter to be pulled therewith, or at best only a small fragment of thrombus to enter this space which in practice will be insufficient to pull the remainder of the thrombus with it.

As the device can reach high temperatures, it is preferred that at least the distal end of the catheter is made of a heat resistant material, although in most embodiments it is preferred that the entirety of the catheter is made of a heat resistant material.

The skilled person will appreciate that where there is no electrical circuit generated at the distal end of the catheter, it is not necessary for the catheter to be electrically insulating but can be solely heat insulating.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosure in the abstract accompanying this application is incorporated herein by reference.

## Claims

1. Medical ablation apparatus including:
an endoluminal delivery catheter having at least one lumen therein, the lumen having a transverse size and cross-sectional shape, the catheter having an outer surface and a distal end wall, the outer surface extending to the distal end wall; and
an elongate flexible ablation device having a transverse size and cross-sectional shape substantially conforming to the transverse size and cross-sectional shape of the catheter lumen, the elongate ablation device having a blunt distal end, the elongate ablation device being slidably received in the catheter;
an adjustment device for retracting the elongate ablation device into the catheter;
wherein at least the distal end wall of the catheter is isolated from the elongate ablation device and provides a rigid insulated detachment shoulder.

2. Medical ablation apparatus according to claim 1, wherein the apparatus is an RF ablation apparatus and the elongate ablation device includes at least one electrically conductive terminal forming part of an electrical circuit of the ablation apparatus; wherein at least the distal end wall of the catheter is isolated from the elongate ablation device and provides an electrically insulated detachment shoulder.

3. Medical ablation apparatus according to claim 2, wherein the electrically conductive terminal is a flexible wire.

4. Medical ablation apparatus according to claim 2 or 3, including a length of insulation sleeve over a part of the electrically conductive terminal, disposed adjacent the end wall of the catheter.

5. Medical ablation apparatus according to any preceding claim, wherein the elongate ablation device includes a resistive heating element, a heat conductive probe, a hot copper wire, an optical fibre for delivery laser energy or a microwave antenna.

6. Medical ablation apparatus according to any preceding claim, wherein the catheter lumen and the elongate ablation device are substantially circular in transverse cross-section.

7. Medical ablation apparatus according to any preceding claim, wherein the transverse size of the lumen is no more than 0.1 millimetres greater than the transverse size of the elongate ablation device.

8. Medical ablation apparatus according to any preceding claim, wherein the distal end wall of the catheter has a diameter at least two times the diameter of the elongate ablation device.

9. Medical ablation apparatus according to any preceding claim, wherein the distal end wall is disposed along a transverse plane of the catheter.

10. Medical ablation apparatus according to any preceding claim, wherein:
a) the catheter is entirely electrically insulating at all exposed surfaces thereof;
b) at least the distal end of the catheter is electrically non-conducting, and/or
c) the end wall is made of conductive material electrically insulated from the elongate ablation device.

11. Medical ablation apparatus according to any one of claims 1 to 9, wherein at least the end wall is made of an insulating material.

12. Medical ablation apparatus according to claim 11, wherein at least the end wall is made of polytetrafluoroethylene (PTFE).

13. Medical ablation apparatus according to claim 12, wherein the distal end wall includes an inflatable balloon.

14. Medical ablation apparatus according to any preceding claim, wherein the distal end wall of the catheter is radiopaque.

15. Medical ablation apparatus according to any preceding claim, wherein the elongate ablation device includes and electrical terminal forming part of a monopolar ablation device.
